(19) Europäisches Patentamt
European Patent Office
Office européen des brevets

(11) Numéro de publication: **0 235 043**
**B1**

(12) **FASCICULE DE BREVET EUROPEEN**

(45) Date de publication du fascicule du brevet: **23.01.91**

(21) Numéro de dépôt: **87400405.4**

(22) Date de dépôt: **24.02.87**

(51) Int. Cl.$^5$: **C 07 D 498/06,** A 61 K 31/535 // A61K31/40 ,(C07D498/06, 265:00, 209:00)

(54) **Dérivés de tétrahydrobenz (c,d) indole, leurs sels, procédé de préparation, application à titre de médicaments, compositions les renfermant et intermédiaires.**

(30) Priorité: **24.02.86 FR 8602509**

(43) Date de publication de la demande:
**02.09.87 Bulletin 87/36**

(45) Mention de la délivrance du brevet:
**23.01.91 Bulletin 91/04**

(84) Etats contractants désignés:
**CH DE GB IT LI NL**

(56) Documents cités:
**EP-A-0 037 784**
**EP-A-0 094 305**

(73) Titulaire: **ROUSSEL-UCLAF**
**35, boulevard des Invalides**
**F-75007 Paris (FR)**

(72) Inventeur: **Nedelec, Lucien**
**45, Boulevard de l'Ouest**
**F-93340 Le Raincy (FR)**
Inventeur: **Rettien, Claude**
**28, rue de La Fédération**
**F-93100 Montreuil-Sous-Bois (FR)**
Inventeur: **Gueniau, Claude**
**175, Avenue Jean Jaurès**
**F-75019 Paris (FR)**

(74) Mandataire: **Vieillefosse, Jean-Claude et al**
**Département des Brevets ROUSSEL UCLAF B.P no 9**
**F-93230 Romainville (FR)**

**EP 0 235 043 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne des nouveaux dérivés de tétrahydrobenz (c, d) indole ainsi que leurs sels, le procédé de préparation, l'application à titre de médicaments de ces dérivés, les compositions les renfermant, et des intermédiaires.

Les brevets européens EP—A—0037784 et EP—A—0094305 concernent des dérivés de l'oxaergoline différents de produits de la présente demande.

L'invention a pour objet des nouveaux dérivés de tétrahydrobenz (c, d) indole ainsi que leurs sels d'addition avec les acides minéraux ou organiques, caractérisés en ce qu'ils répondent à la formule générale (I):

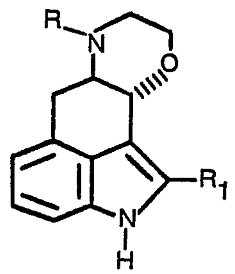

(I)

dans laquelle R représente un radical alkyle renfermant de 1 à 6 atomes de carbone, linéaire, ramifié ou cyclisé, ou un atome, d'hydrogène, et $R_1$ représente un atome d'hydrogène, de chlore ou de brome.

Dans la formule générale (I) et dans ce qui suit, le terme radical alkyle linéaire renfermant de 1 à 6 atomes de carbone désigne, de préférence, un radical méthyle, éthyle ou n-propyle, ramifié de préférence un radical isopropyle ou isobutyle, et cyclisé de préférence un radical cyclopropylméthyle ou cyclohexyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits objet de l'invention, on peut citer notamment les dérivés répondant à la formule (I) ci-dessus, ainsi que leurs sels d'addition avec les acides minéraux ou organiques, caractérisés en ce que, dans ladite formule (I), $R_1$ représente un atome d'hydrogène.

Parmi ceux-ci, on peut citer plus particulièrement les dérivés caractérisés en ce que dans ladite formule (I), R représente un atome d'hydrogène ou un radical alkyle linéaire renfermant de 1 à 6 atomes de carbone et tout particulièrement:

la (d,l-trans) 2,6a, 7, 8, 9, 10a-hexahydro 7-propyl 6H-indolo/4,3-gh/1,4-benzoxazine ainsi que ses d'addition avec les acides minéraux ou organiques.

On peut citer également:

la (d,l-trans) 1-bromo 2, 6a, 7, 8, 9, 10a-hexahydro 7-propyl 6H-indolo/4,3-gh/1,4-benzoxazine.

L'invention a également pour objet un procédé de préparation des dérivés tels que définis par la formule I ci-dessus, ainsi que de leurs sels, caractérisés en ce que l'on réduit un dérivé de formule II:

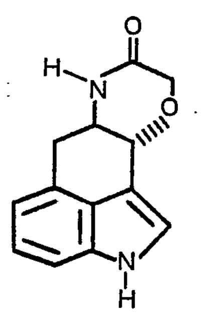

(II)

pour obtenir un produit de formule $I_A$:

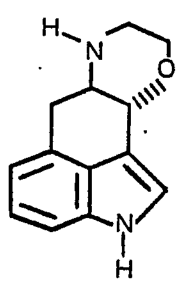

($I_A$)

que soit l'on isole et, si désiré, salifie, soit l'on soumet à l'action d'un agent d'alkylation, pour obtenir un produit de formule $I_B$:

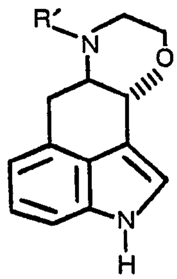

($I_B$)

dans laquelle R' a la signification déjà indiquée pour R, à l'exception de l'hydrogène, que l'on isole et, si désiré, salifie, puis soumet si désiré lesdits produits de formule $I_A$ et $I_B$ à l'action d'un agent d'halogénation pour obtenir un produit de formule $I_C$:

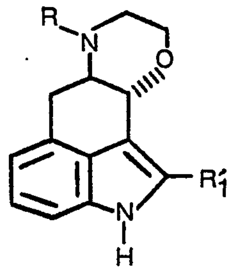

($I_C$)

dans laquelle R a la signification déjà indiquée, et $R'_1$ a la signification de $R_1$ déjà indiquée, à l'exception de l'hydrogène, que l'on isole et, si désiré, salifie.

La réduction des dérivés de formule II est réalisée de préférence par action d'un hydrure tel que l'hydrure d'aluminium-lithium.

L'agent d'alkylation est de préférence un halogénure d'alkyle, notamment un iodure d'alkyle, que l'on fait avantageusement réagir en présence d'une agent de condensation tel qu'une base comme un carbonate ou bicarbonate alcalin. Pour la méthylation, on opère avantageusement par action de formol en présence d'un agent réducteur tel que le borohydrure, et notamment le cyano borohydrure de potassium ou de sodium.

L'agent d'halogénation que l'on fait réagir sur le dérivé de formule $I_B$ peut être, par exemple, la N-chloro succinimide dans le cas de la chloruration, le N-bromo succinimide ou de préférence le complexe bromé de la pyrrolidone de formule:

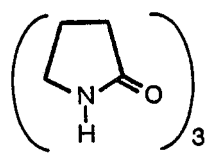

$Br_2$, HBr dans le cas de la bromuration.

Les dérivés de formule II peuvent être préparés comme suit:

On fait réagir le 4-formyl-indole avec le chlorure de triphényl phosphonium N,N-diméthyl acétamide pour obtenir un dérivé de formule III:

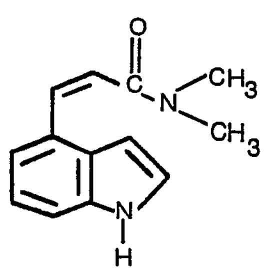

(III)

que l'on soumet à une hydrogénation pour obtenir un dérivé de formule IV:

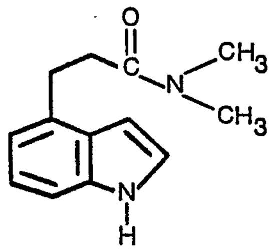

(IV)

que l'on cyclise pour obtenir un dérivé de formule V:

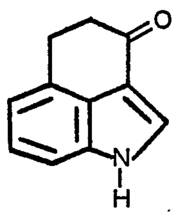

(V)

que l'on bromure pour obtenir un dérivé de formule VI:

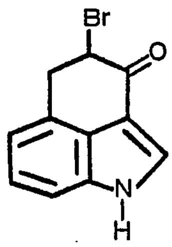

(VI)

que l'on fait réagir avec un azidure pour obtenir un dérivé de formule VII:

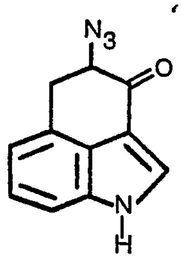

(VII)

que l'on soumet à une hydrogénation pour obtenir un dérivé de formule VIII:

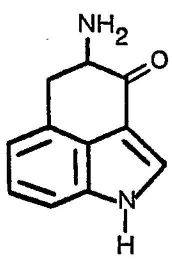

(VIII)

que l'on fait réagir avec le chlorure de de chloroacétyle pour obtenir un dérivé de formule IX:

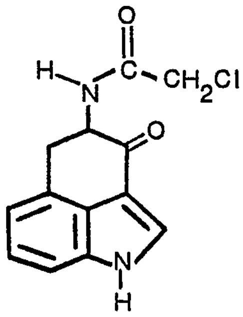

(IX)

que l'on réduit pour obtenir un dérivé de formule X:

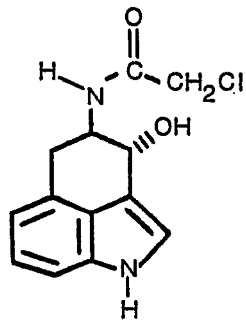

(X)

que l'on cyclise pour obtenir le dérivé de formule II cherché.

La réaction du 4-formyl indole avec le chlorure de triphényl phosphonium N,N-diméthyl acétamide est réalisée de préférence dans les conditions habituelles de la réaction de Wittig.

L'hydrogénation du dérivé de formule III est réalisée de préférence en présence d'un catalyseur, tel que le palladium, le platine ou le nickel de Raney. On opère dans un solvant tel qu'un alcanol de faible poids moléculaire comme le méthanol ou l'éthanol, ou dans un solvant inerte tel que le dioxanne.

La cyclisation du dérivé de formule IV est réalisée de préférence en présence d'oxychlorure de phosphore. On effectue ensuite une hydrolyse alcaline, par exemple par la soude ou de préférence l'ammoniaque aqueuse.

La bromuration du dérivé de formule V est réalisée de préférence à l'aide de brome. On peut utiliser également des réactifs tel que le perbromure de pyridinium ou le N-bromo succinimide.

L'azidure est de préférence un azidure alcalin, de préférence de sodium. On peut utiliser d'autres azidures tels que l'azidure de triméthylsilyle.

L'hydrogénation du dérive de formule VII est réalisée de préférence dans les mêmes conditions que celles utilisées pour le dérivé de formule III.

La réaction du dérivé de formule VIII avec le chlorure de chloroacétyle est réalisée de préférence en présence d'un agent fixateur d'acides, de préférence l'hydroxyde de sodium, dans un solvant organique tel que le dioxanne ou le tétrahydrofuranne et, de préférence, le chloroforme.

La réduction du dérivé de formule IX est réalisée de préférence à l'aide d'un hydrure tel un cyanoborohydrure, et notamment un borohydrure alcalin tel que le borohydrure de sodium.

La cyclisation du dérivé de formule X est réalsiée par exemple à l'aide d'une base forte tel qu'un alcoolate alcalin, tel l'éthylate ou le terbutylate de sodium, mais de préférence à l'aide d'hydrure de sodium. Elle est avantageusement réalisée dans un éther tel que le dioxanne ou le tétrahydrofuranne, mais de préférence dans le 1,2-diméthoxyéthane.

Les dérivés de formule (I) préentent un caractère basique. On peut avantageusement préparer les sels d'addition des dérivés de formule (I) en faisant réagir, en proportions sensiblement stoechiométriques, un acide minéral ou organique avec lesits dérivés de formule (I). Les sels peuvent être préparés sans isoler les bases correspondantes.

Les dérivés, objet de la présente invention, possèdent de très intéressantes propriétés pharmacologiques, ils sont doués notamment de remarquables propriétés agonistes dopaminergiques, hypotensives et antihypertensives. Ils sont, par ailleurs, doués de propriétés antianoxiques.

Ces propriétés sont illustrées plus loin dans la partie expérimentale.

Ces propriétés justifient l'utilisation des dérivés de tétrahydrobenz (c, d) indole, ainsi que de leurs sels, à titre de médicaments.

La présente demande a ainsi également pour objet l'application, à titre de médicaments, des dérivés de tétrahydrobenz (c, d) indole, tels que définis par la formule (I), ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés de tétrahydrobenz (c, d) indole répondant à la formule (I) dans laquelle $R_1$ représente un atome d'hydrogène ainsi que par leurs sels d'addition avec les acides pharmaceutiquement acceptables.

Parmi ceux-ci, on retient notamment ceux répondant à la formule (I), dans laquelle R représente un atome d'hydrogène ou un radical alkyle linéaire renfermant de 1 à 6 atomes de carbone ainsi que leurs sels d'addition avec les acides pharmaceutiquement acceptables.

Parmi ces derniers, on retient tout particulièrement, le dérivé dont le nom suit:

la (d,l-trans) 2,6a, 7, 8, 9, 10a-hexahydro 7-propyl 6H-indolo/4,3-gh/1,4-benzoxazine ainsi que ses d'addition avec les acides pharmaceutiquement acceptables.

Les médicaments selon l'invention trouvent leur emploi, par exemple dans le traitement des syndromes neurologiques d'origine extrapyramidale, par exemple dans le traitement de la maladie de Parkinson et dans le traitement des syndromes parkinsonniens post-encéphalitiques. Ils peuvent aussi être utilisés dans la traitement de l'hypertension artérielle essentielle, de l'hypertension de la cinquantaine, de

la ménopause, du diabétique, de l'obèse et du pléthorique, ainsi que dans le traitement de l'hypertension artérielle du sujet âgé ou atteint d'artériosclérose, et dans le traitement de l'hypertension d'origine rénale. Ils peuvent aussi être utilisés dans le traitement de la sénescence cérébrale ou des manifestations liées à un hypoxie cérébrale.

La dose usuelle, variable selon le dérivé utilisé, le sujet et l'affection en cause peut être par exemple, de 1 mg à 200 mg par jour. Par voie orale, chez l'homme, le dérivé de l'exemple 2 peut être administré à la dose quotidienne de 1 mg à 100 mg, par exemple pour le traitement de la sénescence cérébrale, soit environ de 0,015 mg à 1,5 mg par kilogramme de poids corporel.

L'invention a également pour objet les compositions pharmaceutiques qui renferment au moins un dérivé précité ou l'un de ses sels d'addition avec les acides pharmaceutiquement acceptables, à titre de principe actif.

A tire de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacoa, les véhicules aqueux ou non, les corps gras d'origine animale ou végétale, les dérivés paraffiniques, les glycols, les divers agents mouillants, dispersants ou émulsifiants, les conservateurs.

L'invention s'étend en outre aux produits industriels nouveaux utiles notamment pour la préparation des dérivés répondant à la formule (I), à savoir les dérivés de formule (II):

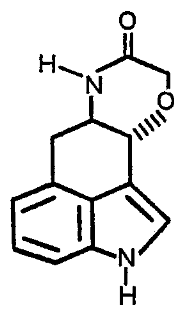

(II)

Les exemples qui suivent illustrent la présente invention, sans toutefois la limiter.

Exemple N° 1

(dl-trans) 2,6,6a,7,8,9,10a-hexahydro 6H-indolo/4,3-gh/1,4-benzoxazine et son (E) 2-butène dioate (2:1)

On porte sous agitation à 60°C sous atmosphère inerte pendant 4 heures 1,4 g de (E) 2,6a,7,10a-tétrahydro 6H-indolo/4,3-gh/1,4-benzoxazin-8(9H)-one et 700 mg d'hydrure d'aluminium lithium dans 15 $cm^3$ de tétrahydrofuranne. On ajoute 5 $cm^3$ de tétrahydrofuranne hydraté à 10% en refroidissant, extrait au chlorure de méthylène, sèche, amène à sec sous pression réduite, purifie par chromatographie sur silice (Eluant: chlorure de méthylène-méthanol 9—1) et obtient 540 mg du produit attendu. Formation du (E) 2-butène dioate (2:1):

On ajoute 60 $cm^3$ de méthanol à 1 g de base ci-dessus, filtre, ajoute 290 mg d'acide fumarique, puis 50 $cm^3$ d'acétate d'éthyle, essore et recristallise dans le méthanol et obtient 850 mg du produit attendu F° ≃ 255°C.

Spectre UV (Méthanol + Diméthylsulfoxyde)

| | | | |
|---|---|---|---|
| Max | 273 nm | $E_1^1$ = 254 | ε = 6.900 |
| Max | 279 " | $E_1^1$ = 259 | ε = 7.050 |
| Max | 289–290 nm | $E_1^1$ = 206 | ε = 5.600 |

Exemple N° 2

(dl-trans) 2,6a,7,8,9,10a-hexahydro 7-propyl 6H-indolo/4,3-gh/1,4-benzoxazine et son (E) butène dioate (2:1)

On agite pendant 1 heure à 50°C sous atmosphère inerte 439 mg de 40 base de l'exemple 1,4 $cm^3$ de diméthylformamide, 553 mg de carbonate de potassium et 1 $cm^3$ d'iodure de propyle. On verse sur 50 $cm^3$ d'eau, extrait à l'acétate d'éthyle, sèche, amène à sec sous pression réduite, et obtient 412 mg du produit attendu.

Formation du (E) butène dioate (2:1)

On dissout la base ci-dessus dans 5 $cm^3$ de méthanol, ajoute 93 mg d'acide fumarique, 20 $cm^3$ d'acétate d'éthyle, concentre à 5 $cm^3$ environ, essore et obtient 300 mg du produit attendu F° ≃ 260°C.

Spectre UV (Ethanol HCl 0,1 N)

| | | | |
|---|---|---|---|
| Max | 220 nm | $E_{11}$ = 1.268 | ε = 39.900 |
| Max | 272 nm | $E_{11}$ = 191 | ε = 6.000 |
| Max | 278 nm | $E_{11}$ = 195 | ε = 6.100 |
| Max | 290 nm | $E_{11}$ = 160 | ε = 5.000 |

Exemple N° 3

(dl-trans) 2,6a,7,8,9,10a-hexahydro 7-méthyle 6H-indol/4,3-gh/1,4-benzoxazine et son chlorhydrate

On agite pendant 1 heure sous atmosphère inerte 1 g de base de l'exemple 1 avec 4 $cm^3$ de formaldéhyde à 40%, 20 $cm^3$ de méthanol et 950 mg de cyanoborohydrure de sodium, ajoute 50 $cm^3$ d'eau, extrait à l'acétate d'éthyle, sèche, amène à sec sous pression réduite, et obtient 900 mg du produit attendu.

Formation du chlorhydrate:

On salifie la base ci-dessus sous forme de chlorhydrate, la recristallise dans le méthanol et obtient 700 mg du produit attendu F° ≃ 260°C.

Spectre UV (Ethanol HCl)

| | | | |
|---|---|---|---|
| Max | 220 nm | $E_{11}$ = 1.279 | ε = 36.600 |
| Max | 220 nm | $E_{11}$ = 212 | ε = 6.100 |
| Max | 279 nm | $E_{11}$ = 222 | ε = 6.350 |
| Infl | 282 nm | $E_{11}$ = 205 | |
| Max | 290 nm | $E_{11}$ = 181 | ε = 5.200 |

Préparation de la (E) 2,6a,7,10a-tétrahydro 6H-indol/4,3 gh/benzoxazine 8(9H)-one:

Stade A:

N,N-diméthyl 3-(1H-indol 4-yl) 2-propénamide

On agite pendant 19 heures, 6 g de 4-formyl indole dans 100 $cm^3$ de tétrahydrofuranne, avec, l'ylide corresponant au triphénylphosphonium N,N-diméthyl acétamide préparé comme suit:

On porte au reflux pendant 19 heures une solution de 15,6 g de triphénylphosphine et de 6 g de 2-chloro N,N-diméthylacétamide dans 100 $cm^3$ de nitrométhane, amène à sec, lave à l'éther isopropylique, sèche sous pression réduite et obtient 21 g de cristaux incolores dont on dissout 11 g dans 10 $cm^3$ d'eau, alcalinise la solution à la soude 2N, extrait au chlorure de méthylène, sèche, filtre et amène à sec sous pression réduite pour obtenir l'ylide recherché.

Après les 19 heures, on chromatographie sur silice (Eluant:cyclohexane — acétate d'éthyle 5—5) et obtient 7 g du produit attendu F° ≃ 185°C.

Stade B:

N,N-diméthyl 1H-indol 4-yl) 4-propanamide

On hydrogène pendant 4 heures 30 à pression normale 187 g de produit du stade A dans 1,5 l d'éthanol en présence de 9 g de palladium à 10% sur charbon actif, filtre, lave à l'aide d'1 l de chloroforme, amène à sec sous pression réduite, empâte dans l'éther, filtre, sèche sous pression réduite et obtient 156,3 g du produit attendu F° ≃ 118°C.

Stade C:

4,5-dihydro benz (c, d) indol 3(1H)-one

On agite à 70°C sous atmosphère inerte, pendant 3 heures, 50 g de produit du stade B dans 500 $cm^3$ de tétrahydrofuranne et 125 $cm^3$ d'oxychlorure de phosphore, verse sur 1,8 l d'ammoniaque 5 N à 5°C, extrait à l'acétate d'éthyle, lave à l'eau, sèche, amène à sec sous pression réduite, purifie par chromatographie sur silice (Eluant: cyclohexane-acétate d'éthyle 7—3) et obtient 19,2 g du produit attendu.

F° ≃ 184°C.

Stade D:

4-bromo 4,5-dihydro benz (c, d) indol 3(1H)-one

On refroidit à −17°C une solution de 5 g de produit de stade C dans 50 $cm^3$ de tétrahydrofuranne sous

atmosphère inerte, ajoute 50 $cm^3$ d'une solution à 20% de brome dans le tétrahydrofuranne, verse sur 400 $cm^3$ d'eau, extrait au chlorure de méthylène, sèche, amène à sec sous pression réduite, cristallise dans l'éther isopropylique, et obtient 5,4 g du produit attendu.

F° ≃ 210°C.

Stade E:

4-azido 4,5-dihydro benz (c, d) indol 3(1H)-one

On agite pendant 15 heures à 5°C une solution de 6,7 g d'azidure de sodium et 11,5 g de produit du stade D dans 115 $cm^3$ de diméthyl formamide, verse sur 500 $cm^3$ d'eau, essore le produit obtenu, purifie par chromatographie sur silice (Eluant: cyclohexane-acétate d'éthyle 5—5) et obtient 7 g du produit attendu

F° ≃ 150°C avec décomposition.

Stade F:

4-amino 4,5-dihydro benz (c, d) indol 3(1H)-one

On hydrogène pendant 6 heures sous agitation, 6 g de produit du stade E dans 200 $cm^3$ d'éthanol en présence de 600 mg de palladium à 10% sur charbon actif, filtre, lave à l'aide de chloroforme, amène le filtrat à sec sous pression réduite et obtient 6 g de produit attendu

F° ≃ 225°C.

Stade G:

2-chloro N-(3-oxo 1,3,4,5-tétrahydro benz (c,d) indol 4-yl) acétamide.

On agite pendant 30 mn dans un bain de glace 850 mg de produit du stade F, 39 $cm^3$ de chloroforme, 0.75 $cm^3$ de chlorure de monochloroacétyle et 10 $cm^3$ d'eau, filtre, lave à l'eau le produit cristallisé, le sèche et obtient 800 mg du produit attendu.

Stade H:

2-chloro N-(3-hydroxy 1,3,4,5-tétrahydro benz (c,d) indol 4-yl) acétamide.

On agite pendant 30 mn 800 mg de produit du stade G dans 8 $cm^3$ de méthanol et 8 $cm^3$ d'eau avec 400 mg de borohydure de sodium extrait à l'acétate d'éthyle, lave à l'eau, sèche, amène à sec sous pression réduite; purifie par chromatographie sur silice (Eluant:Acétate d'éthyle) et obtient 520 mg du produit attendu.

Stade I:

(E) 2,6a,7,10a-tétrahydro 6H-indolo/4,3 gh/1,4-benzoxazin 8(9H)-one

On agite pendant 30 mn dans un bain de glace 1,5 g de produit du stade H dans 30 $cm^3$ de 1,2-diméthoxy éthane avec 330 mg d'hydrure de sodium à 55% dans l'huile, ajoute à nouveau 330 mg d'hydrure de sodium, après 2 heures 30, ajoute avec précaution 200 $cm^3$ d'eau, filtre, lave à l'eau, essore, sèche sous pression réduite et obtient 900 mg de produit attendu.

F° > 260°C.

Spectre UV :

| | | | |
|---|---|---|---|
| Max | 221 nm | $E_1^1$ = 1.422 | $\varepsilon$ = 32.500 |
| Max | 272 " | $E_1^1$ = 260 | $\varepsilon$ = 5.900 |
| Max | 279 " | $E_1^1$ = 263 | $\varepsilon$ = 6.000 |
| Max | 290 " | $E_1^1$ = 216 | $\varepsilon$ = 4.900 |
| Infl | 320,330,360 nm | | |

Exemple N° 4

On a préparé des comprimés répondant à la formule:

sel de l'exemple 2:

| | |
|---|---|
| (E) butène dioate de (d,l trans) 2,6a,7,8,9,10a hexahydro 7-propyl 6H-indolo/4,3-g,h/1,4-benzoxazine | 2 mg; |
| Excipient q.s. pour un comprimé terminé à | 100 mg. |

(Détail de l'excipient: lactose, amidon, talc, stéarate de magnésium).

Exemple N° 5

On a préparé des comprimés répondant à la formule:

sel de l'exemple 3:

| | |
|---|---|
| Chlorhydrate de (d,l trans) 2,6a,7,8,9,10a-hexahydro 7-méthyl 6H-indolo/4,3-g,h/1,4-benzoxazine | 10 mg; |
| Excipient q.s. pour un comprimé terminé à | 100 mg. |

(Détail de l'excipient:lactose, amidon, talc, stéarate de magnésium).

Etude pharmacologique

1/. Affinités pour les récepteurs dopaminergiques

On homogénéise au vingtième (poids/volume) dans le sucrose 0.32M, les corps striés prélevés des cerveaux de 6 râts mâles pesant 150 g en moyenne. Après centrifugation du mélange homogénéisé à 1000 g pendant 10 mn à 0°C, le surnageant est centrifugé à 30.000 g pendant 15 mn à +4°C. Le culot et repris dans 25 ml de tampon Tris HCl 50 mM pH 7,7 et centrigé à 30.000 g pendant 15 mn à +4°C . Le nouveau culot est repris dans 50 ml de tampon Krebs Tris HCl pH 7,3 et la suspension est préincubée pendant 10 mn à 37°C. On fait ensuite incuber pendant 29 mn au bain-marie à +37°C en présence de spinopéridol $^3$H, seul, avec un excès d'halopéridol, et avec des concentrations croissantes du produit à tester. Les suspensions incubées sont filtrés sur Whatman GF/C et les filtres sont lavés par trois fois 5 ml de tampon Tris HCl 50 mM. La radioactivité des filtres est mesurée par scintillation liquide.

La fixation non spécifique est déterminée parallèlement par incubation de spiropéridol $^3$H en présence d'un excès d'halopéridol.

L'affinité du produit testé pour les récepteurs dopaminergiques est donnée relativement à l'halopéridol comme produit de référence.

CD = Concentration d'halopéridol froid inhibant 50% de la fixation spécifique du spiropéridol $^3$H,

CX = Concentration du produit à tester inhibant 50% de la fixation spécifique du spiropéridol $^3$H.

L'affinité relative est donnée par la relation:

$$ARL = 100 \frac{CD}{CX}$$

On a obtenu les résultats suivants:

| PRODUIT TESTE | AFFINITE RELATIVE (HALOPERIDOL = 100) |
|---|---|
| Exemples : 1 | 0,09 |
| 2 | 13 |
| 3 | 0,4 |

2/. Détermination de l'activité hypotensive.

L'activité hypotensive a été étudiée sur des rats mâles de souche WISTAR pesant 300 g environ et anexthésiés au nembutal (50 mg/Kg par voie intrapéritonéale).

Le produit testé a été administré par voie intraveineuse dans la veine jugulaire.

La pression artérielle carotidienne a été mesurée avant et après administration du produit testé.

Le tableau ci-après indique les variations exprimées en pourcentage de la pression artérielle après administration du produit testé par rapport à la pression artérielle témoin initiale.

| Produit de l'exemple | Dose mg/kg | Variation % de la pression artérielle |
|---|---|---|
| | | 5 minutes après l'administration |
| 1 | 1 | Variation de la pression artérielle comprise entre -30% et -40% |
| 2 | 0,1<br>0,01 | Variation de la pression artérielle au delà de -40% |

3/. Activité antianoxique

a — Test de l'anoxie hypobare:

On utilise des ouris mâles d'un poids de 20—22 g, à jeun depuis cinq heures, réparties par groupe de 10 animaux.

On administre aux animaux le produit à tester, par voie sous-cutanée. Quinze minutes après administation du produit, on place les animaux dans un dessicateur de 2 litres dans lequel on amène rapidement la pression à 0,253 bar (190 mm de Hg), au moyen d'une pompe, et on note leur temps de survie exprimé en secondes.

On calcule l'augmentation du temps de survie des animaux traités par rapport aux animaux témoins, soumis aux mêmes conditions.

Les produits des exemples 1, 2 et 3 sont actifs respectivement dès la dose de 1 mg/Kg, 0,01 mg/Kg et 1 mg/Kg.

b — test de l'énolase:

Les cellules cérébrales en souffrance libèrent de l'énolase qui est un marqueur spécifique de lésion neuronale. Dans le test, les lésions sont réalisées chez la souris par injection SC de 35 mg/Kg d'acide kaïnique. Le produit de l'exemple n° 2 administré par voie intrapéritonéale, dès la dose de 1 mg/Kg, 1 heure avant l'injection du neurotoxique, diminue la concentration sérique d'énolase. Par conséquent, ce produit protège les cellules cérébrales en situation de souffrance.

4/. Etude de la toxicité aigüe

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants:

| Produit de l'exemple | $DL_0$ en mg/kg |
|---|---|
| 1 | 200 |
| 2 | 100 |

**Revendications**

1. Les dérivés de tétrahydrobenz (c, d) indole ainsi que leurs els d'addition avec les acides minéraux ou organiques, caractérisés en ce qu'ils répondent à la formule générale (I):

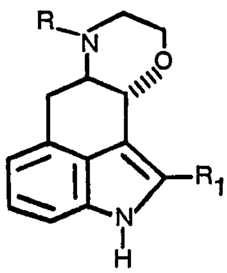

(I)

dans laquelle R représente un radical alkyle renfermant de 1 à 6 atomes de carbone, linéaire, ramifié ou cyclisé, ou un atome, d'hydrogène, et $R_1$ représente un atome d'hydrogène, de chlore ou de brome.

2. Les dérivés tels que définis par la formule I de la revendication 1, ainsi que leurs sels s'addition avec les acides minéraux ou organiques, caractérisés en ce que $R_1$ représente un atome d'hydrogène.

3. Les dérivés selon la revendication 2, ainsi que leurs sels d'addition avec les acides minéraux ou organiques, caractérisés en ce que R représente un atome d'hydrogène ou un radical alkyle linéaire renfermant de 1 à 6 atomes de carbone.

4. Le dérivé dont le nom suit:

la (d,l-trans)-2,6a,7,8,9,10a-hexahydro 7-propyl 6H-indolo/4,3-gh/1,4-benzoxazine ainsi que ses d'addition avec les acides minéraux ou organiques.

5. Procédé de préparation des dérivés tels que définis par la formule I de la revendication 1, ainsi que de leurs sels, caractérisés en ce que l'on réduit un dérivé de formule II:

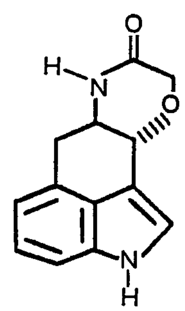

(II)

pour obtenir un produit de formule $I_A$:

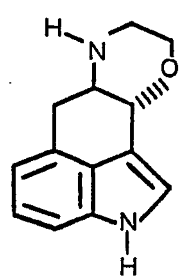

($I_A$)

que soit l'on isole et, si désiré, salifie, soit l'on soumet à l'action d'un agent d'alkylation, pour obtenir un produit de formule $I_B$:

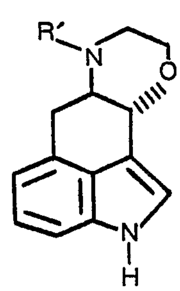

($I_B$)

dans laquelle R' a la signification déjà indiquée pour R, à l'exception de l'hydrogène, que l'on isole et, si désiré, salifie, puis soumet si désiré lesdits produits de formule $I_A$ et $I_B$ à l'action d'un agent d'halogénation pour obtenir un produit de formule $I_C$:

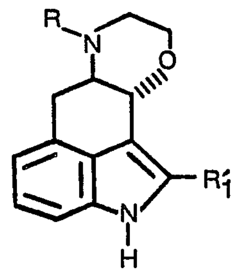

($I_C$)

dans laquelle R a la signification déjà indiquée, et $R'_1$ a la signification de $R_1$ déjà indiquée, à l'exception de l'hydrogène, que l'on isole et, si désiré, salifie.

6. Médicaments, caractérisés en ce qu'ils sont constitués par les nouveaux dérivés de tétrahydrobenz (c, d) indole, tels que définis par la formule I de la revendicaton 1, ainsi que par leurs sels d'addition avec les acides pharmaceutiquement acceptables.

7. Médicaments, caractérisés en ce qu'ils sont constitués par les nouveaux dérivés de tétrahydrobenz (c, d) indole, tels que définis dans l'une des revendications 2 ou 3, ainsi que par leurs sels d'addition avec les acides pharmaceutiquement acceptables.

8. Médicaments, caractérisé en ce qu'ils sont constitués par le dérivé de tétrahydrobenz (c, d) indole, tels que défini à la revendication 4 ainsi que par ses sels d'addition avec les acides pharmaceutiquement acceptables.

9. Compositions pharmaceutiques, caractérisées en ce qu'elles renferment, à titre de principe actif, l'un au moins des médicaments, tels que définis à l'une des revendications 6, 7 ou 8.

10. Le dérivé de formule II:

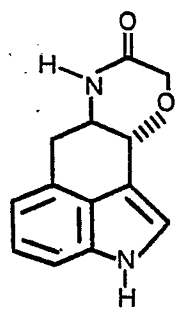

(II)

**Patentansprüche**

1. Tetrahydrobenz(c,d)indol-Derivate sowie deren Additionssalze mit Mineral- oder organischen Säuren, dadurch gekennzeichnet, daß sie der allgemeinen Formel I

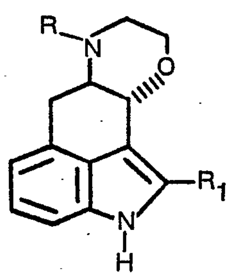

(I)

entsprechen, worin R für eine lineare, verzweigte oder cyclische Alkylgruppe mit 1 bis 6 Kohlenstoffatomen oder ein Wasserstoffatom steht, und $R_1$ ein Wasserstoff-, Chlor- oder Bromatom bedeutet.

2. Derivate der Formel I gemäß Anspruch 1 sowie deren Additionssalze mit Mineral- oder organischen Säuren, dadurch gekennzeichnet, daß $R_1$ für ein Wasserstoffatom steht.

3. Derivate gemäß Anspruch 2 sowie deren Additionssalze mit Mineral- oder organischen Säuren, dadurch gekennzeichnet, daß R für ein Wasserstoffatom oder eine lineare Alkylgruppe mit 1 bis 6 Kohlenstoffatomen steht.

4. Derivat mit der folgenden Bezeichnung: (d,l-trans)-2,6a,7,8,9,10a-Hexahydro-7-propyl-6H-indolo-(4,3-gh)1,4-benzoxazin sowie dessen Additionssalze mit Mineral- oder organischen Säuren.

5. Verfahren zur Herstellung der Drivate der Formel I gemäß Anspruch 1 sowie von deren Salzen, dadurch gekennzeichnet, daß man ein Derivat der Formel II

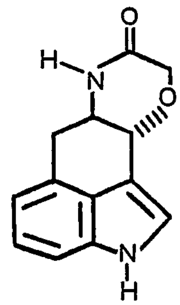

(II)

reduziert, um zu einem Produkt der Formel $I_A$

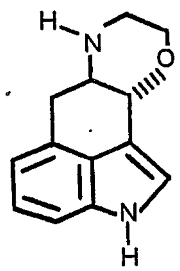

($I_A$)

zu gelangen, welches man entweder isoliert und gewünschtenfalls in ein Salz überführt oder der Einwirkung eines Alkylierungsmittels unterzieht, um zu einem Produkt der Formel $I_B$

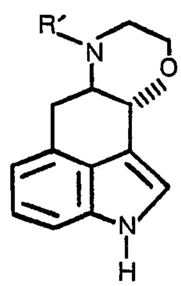

($I_B$)

worin R′ die für R angegebene mit Ausnahme von Wasserstoff besitzt, zu gelangen, welches man isoliert und gewünschtenfalls in ein Salz überführt, und hiernach gewünschtenfalls die Produkte der Formel $I_A$ und $I_B$ der Einwirkung eines Halogenierungsmittels unterzieht, um zu einem Produkt der Formel $I_C$

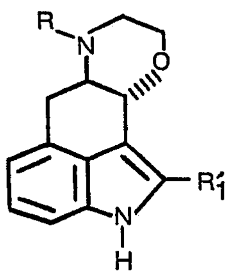

($I_C$)

worin R die angegebene Bedeutung besitzt und $R'_1$ die für $R_1$ angegebene Bedeutung mit Ausnahme von Wasserstoff besitzt, zu gelangen, welches man isoliert und gewünschtenfalls in ein Salz überführt.

6. Arzneimittel, dadurch gekennzeichnet, daß sie aus den neuen Tetrahydrobenz(c,d)indol-Derivaten der Formel I gemäß Anspruch 1 oder aus deren Additionssalzen mit pharmazeutisch verträglichen Säuren bestehen.

7. Arzneimittel, dadurch gekennzeichnet, daß sie aus den neuen Tetrahydrobenz(c,d)indol-Derivaten gemäß einem der Ansprüche 2 oder 3 oder aus deren Additionssalzen mit pharmazeutisch verträglichen Säuren bestehen.

8. Arzneimittel, dadurch gekennzeichnet, daß sie aus dem Tetrahydrobenz(c,d)indol-Derivat gemäß Anspruch 4 oder dessen Additionssalzen mit pharmazeutisch verträglichen Säuren bestehen.

9. Pharmazeutische Zusammensetzungen, dadurch gekennzeichnet, daß sie als Wirkstoff zumindest eines der Arzneimittel gemäß einem der Ansprüche 6, 7 oder 8 enthalten.

10. Derivat der Formel II

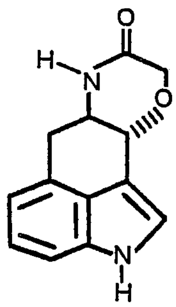

(II)

## Claims

1. The derivatives of tetrahydrobenz (c,d) indole as well as their addition salts with mineral or organic acids, characterized in that they correspond to the general formula (I):

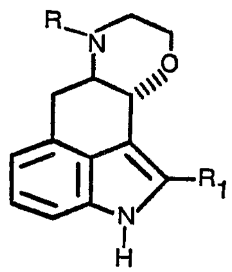

(I)

in which R represents a linear, branched or cyclic alkyl radical containing 1 to 6 carbon atoms, or a hydrogen atoms, and $R_1$ represents a hydrogen, chlorine or bromine atom.

2. The derivatives as defined by formula (I) of claim 1, as well as their addition salts with mineral or organic acids, characterized in that $R_1$ represents a hydrogen atom.

3. The derivatives according to claim 2, as well as their addition salts with mineral or organic acids, characterized in that R represents a hydrogen atom or a linear alkyl radical containing 1 to 6 carbon atoms.

4. The derivative the name of which follows:
(d,l-trans) 2,6a,7,8,9,10a-hexahydro-7-propyl-6H-indolo-[4,3-gh]-1,4-benzoxazine as well as its addition salts with mineral or organic acids.

5. Process for the preparation of derivatives as defined by formula (I) of claim 1, as well as their salts, characterized in that a derivative of formula (II):

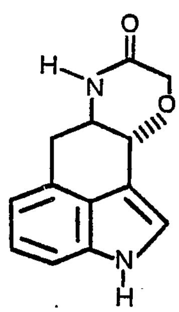

(II)

is reduced to obtain a product of formula ($I_A$):

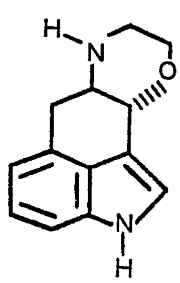

($I_A$)

which either is isolated and, if desired, salified, or is subjected to the action of an alkylation agent, to obtain a product of formula ($I_B$):

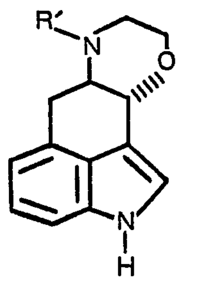

($I_B$)

in which R′ has the meaning already indicated for R, with the exception of hydrogen, which is isolated and, if desired, salified, then if desired the said products of formulae ($I_A$) and ($I_B$) are subjected to the action of a halogenation agent to obtain a product of formula ($I_C$):

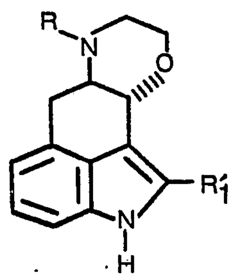

($I_C$)

in which R has the meaning already indicated, and $R'_1$ has the meaning already indicated for $R_1$, with the exception of hydrogen, which is isolated and, if desired, salified.

6. Medicaments, characterized in that they are constituted by the new derivatives of tetrahydrobenz (c,d) indole, as defined by formula (I) of claim 1, as well as by their addition salts with pharmaceutically acceptable acids.

7. Medicaments, characterized in that they are constituted by the new derivatives of tetrahydrobenz (c,d) indole, as defined in one of claims 2 or 3, as well as by their addition salts with pharmaceutically acceptable acids.

8. Medicaments, characterized in that they are constituted by the derivative of tetrahydrobenz (c,d) indole, as defined in claim 4, as well as by its addition salts with pharmaceutically acceptable acids.

9. Pharmaceutical compositions, characterized in that they contain, as active ingredient, at least one of the medicaments as defined in one of claims 6, 7 or 8.

10. The derivative of formula (II):

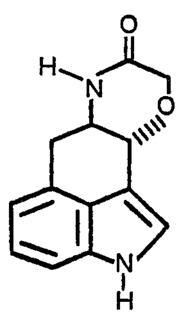

(II)